# EUROPEAN PATENT APPLICATION

(11) **EP 4 506 450 A1**
(43) Date of publication of application: **12.02.2025**
(21) Application number: 23784767.8
(22) Date of filing: 05.04.2023
(51) Int. Cl.: C12N 5/10, C12N 1/15, C12N 1/19, C12N 1/21, C12N 15/55, C12N 15/63, C12P 7/40, C12N 9/16

(54) **MODIFIED ESTERASE**

(30) Priority: 05.04.2022 JP 2022063122
(71) Applicant: Amano Enzyme Inc., Nagoya-shi Aichi 460-8630 (JP)
(72) Inventor: KODAMA, Yusaku, Kakamigahara-shi, Gifu 509-0109 (JP); URANO, Nobuyuki, Kakamigahara-shi, Gifu 509-0109 (JP)
(74) Representative: J A Kemp LLP
(86) International application number: PCT/JP2023/014078
(87) International publication number: WO 2023/195483

(57) **Abstract**

The present invention provides any one of the following modified esterases (i) to (iii):
(i) a modified esterase comprising an amino acid sequence in which at least one amino acid at a site selected from the group consisting of (1) to (24) below in the amino acid sequence of SEQ ID NO: 1 or 3 is substituted with another amino acid, (1) V64 (2) S65 (3) A102 (4) A109 (5) L151 (6) I153 (7) Y173 (8) Y197 (9) L200 (10) I211 (11) F217 (12) S220 (13) D222 (14) D227 (15) L229 (16) L242 (17) A245 (18) V257 (19) L298 (20) T301 (21) V326 (22) S329 (23) A350 (24) T371;
(ii) a modified esterase in which one or more amino acids are further substituted (except for the amino acid site substituted in (i)), added, inserted, or deleted (except for the amino acid site substituted in (i)) in the modified esterase of (i), and the reactivity to (1R,3R)-methyl 3-(2-chloro-3,3,3-trifluoroprop-1-en-1-yl)-2,2-dimethylcyclopropane-1-carboxylate is improved compared to the reactivity of an esterase consisting of the amino acid sequence of SEQ ID NO: 1 or 3;
(iii) a modified esterase in which an amino acid(s) other than the amino acid(s) substituted in (i) is further substituted with another amino acid(s), wherein the modified esterase has 70% or more identity with the modified esterase of (i) and has improved reactivity to (1R,3R)-methyl 3-(2-chloro-3,3,3-trifluoroprop-1-en-1-yl)-2,2-dimethylcyclopropane-1-carboxylate compared to the reactivity of the esterase consisting of the amino acid sequence of SEQ ID NO: 1 or 3.

## Description

### [Technical Field]

The present invention relates to a novel modified esterase. More specifically, the present invention relates to a modified esterase with improved reactivity to a specific substrate.

### [Background Art]

Cyhalothrin acid (hereinafter sometimes referred to as "CA") is used as a raw material for the synthesis of cyhalothrin, a pyrethroid insecticide. There are four types of CA isomers ((1R,3R)-CA, (1R,3S)-CA, (1S,3R)-CA, and (1S,3S)-CA), but cyhalothrin synthesized from (1R,3R)-CA (also called "λ-cyhalothric acid") has the highest insecticidal effect. CA is industrially produced as a mixture of two CA isomers ((1R,3R)-CA and (1S,3S)-CA) using chemical synthesis methods.

For example, Patent Literature 1 discloses that esterase derived from Arthrobacter globiformis decomposes ethyl chrysanthemate in a 1R,3R ((+)-trans) specific manner. Patent Literature 2 discloses a case where esterase derived from Arthrobacter globiformis was modified to an enzyme that decomposes ethyl chrysanthemate in a 1R,3S ((+)-cis) specific manner. Non Patent Literature 1 discloses that esterase derived from Arthrobacter globiformis and esterases of other origins decompose ethyl chrysanthemate in a 1R,3R ((+)-trans) or 1S,3R ((-)-trans) specific manner.

### [Citation List]

### [Patent Literature]

[Patent Literature 1]
   JP H5-56787 A
[Patent Literature 2]
   WO 2020/116331

### [Non Patent Literature]

[Non Patent Literature 1]
Satoshi Mitsuda et al., Agric. Biol. Chem., 55(11), 2865-2870, 1991.

### [Summary of Invention]

### [Technical Problem]

γ-Cyhalothrin is the optically active form with the highest insecticidal effect among the stereoisomers of cyhalothrin. Therefore, it is desirable to selectively produce λ-cyhalothric acid, which is a part of its skeleton. Typical methods for producing λ-cyhalothric acid include, for example, stereoselective hydrolysis using chemical catalysts and purification using a chiral column. However, these methods have problems in terms of yield, cost, and wastewater regulations. Therefore, it is desirable to establish a production method using enzymes that can solve these problems. However, no enzyme has been reported that can efficiently and stereoselectively hydrolyze only the (1R,3R) form of ester compound of λ-cyhalothric acid (methyl cyhalothrate (hereinafter sometimes referred to as "MC")).

The present invention aims to provide a novel esterase that is useful for producing (1R,3R)-CA (λ-cyhalothric acid).

### [Solution to Problem]

The present inventors focused on a quintuple mutant modified esterase of esterase derived from Arthrobacter globiformis reported in Patent literature 2. The esterase had high reactivity to (1R,3S)-ethyl chrysanthemate, and at the same time, low reactivity to (1R,3R)-MC. Therefore, the inventors attempted to improve the reactivity of the esterase to (1R,3R)-MC using protein engineering techniques. After much trial and error, the inventors succeeded in identifying multiple candidate mutation sites in the quintuple mutant modified esterase that are effective in improving the reactivity to (1R,3R)-MC, and further research based on this knowledge led to the completion of the present invention.

That is, the present invention is as follows.

[1] A modified esterase of any of the following (i) to (iii):
   (i) a modified esterase comprising an amino acid sequence in which at least one amino acid at a site selected from the group consisting of (1) to (24) below in the amino acid sequence of SEQ ID NO: 1 or 3 is substituted with another amino acid;
      (1) V64
      (2) S65
      (3) A102
      (4) A109
      (5) L151
      (6) I153
      (7) Y173
      (8) Y197
      (9) L200
      (10) I211
      (11) F217
      (12) S220
      (13) D222
      (14) D227
      (15) L229
      (16) L242
      (17) A245
      (18) V257
      (19) L298
      (20) T301
      (21) V326
      (22) S329
      (23) A350
      (24) T371
   (ii) a modified esterase in which one or more amino acids are further substituted (except for the amino acid site substituted in (i)), added, inserted, or deleted (except for the amino acid site substituted in (i)) in the modified esterase of (i), and the reactivity to (1R,3R)-methyl 3-(2-chloro-3,3,3-trifluoroprop-1-en-1-yl)-2,2-dimethylcyclopropane-1-carboxylate is improved compared to the reactivity of an esterase consisting of the amino acid sequence of SEQ ID NO: 1 or 3;
   (iii) a modified esterase in which an amino acid(s) other than the amino acid(s) substituted in (i) is further substituted with another amino acid(s) in the modified esterase of (i), wherein the modified esterase has 70% or more identity with the modified esterase of (i) and has improved reactivity to (1R,3R)-methyl 3-(2-chloro-3,3,3-trifluoroprop-1-en-1-yl)-2,2-dimethylcyclopropane-1-carboxylate compared to the reactivity of the esterase consisting of the amino acid sequence of SEQ ID NO: 1 or 3.
[2] The modified esterase according to [1], wherein in (i), the amino acid substitution is at least one selected from the group consisting of the following:
   (1) V64M
   (2) S65A
   (3) A102S
   (4) A109T
   (5) L151F, L151M, or L151W
   (6) I153V
   (7) Y173F
   (8) Y197F
   (9) L200F
   (10) 1211V or I211M
   (11) F217K
   (12) S220W
   (13) D222G
   (14) D227C or D227E
   (15) L229G
   (16) L242C, L242I, L242M, or L242W
   (17) A245T
   (18) V257I
   (19) L298H
   (20) T301A
   (21) V326I
   (22) S329D, S329G, or S329N
   (23) A350D
   (24) T371A.
[3] A nucleic acid encoding the modified esterase according to [1] or [2].
[4] An expression cassette or recombinant vector comprising the nucleic acid according to [3].
[5] A transformant transformed using the expression cassette or recombinant vector according to [4].
[6] A method for producing a modified esterase, comprising the step of culturing the transformant according to [5].
[7] An enzyme preparation comprising the modified esterase according to [1] or [2].
[8] The enzyme preparation according to [7], which is used for producing (1R,3R)-3-[(1Z)-2-chloro-3,3,3-trifluoroprop-1-en-1-yl]-2,2-dimethylcyclopropane-1-carboxylic acid.
[9] A method for producing (1R,3R)-3-[(1Z)-2-chloro-3,3,3-trifluoroprop-1-en-1-yl]-2,2-dimethylcyclopropane-1-carboxylic acid, comprising the step of acting the modified esterase described in [1] or [2] on (1R,3R)-methyl 3-(2-chloro-3,3,3-trifluoroprop-1-en-1-yl)-2,2-dimethylcyclopropane-1-carboxylate.

### [Advantageous Effects of Invention]

The modified esterase of the present invention acts with high reactivity on (1R,3R)-MC, and as a result, can efficiently produce (1R,3R)-CA.

### [Description of Embodiments]

Some of the terms used to explain the present invention are explained below.

### <Amino acid>

In the present specification, amino acids are sometimes represented by a single letter, as per convention.
Specifically, it is as follows:
Glycine: G
Alanine: A
Valine: V
Leucine: L
Isoleucine: I
Phenylalanine: F
Tyrosine: Y
Tryptophan: W
Serine: S
Threonine: T
Cysteine: C
Methionine: M
Aspartic acid: D
Glutamic acid: E
Aspartic acid: N
Glutamin: Q
Lysine: K
Arginine: R
Histidine: H
Proline: P

### <Amino acid sequence>

In the the present specification, the amino acid sequence shown is the N-terminus on the left and the C-terminus on the right.

### <Types of amino acids>

In the present specification, "non-polar amino acids" include alanine, valine, leucine, isoleucine, proline, methionine, phenylalanine, and tryptophan. "Uncharged amino acids" include glycine, serine, threonine, cysteine, tyrosine, asparagine, and glutamine. "Acidic amino acids" include aspartic acid and glutamic acid. "Basic amino acids" include lysine, arginine, and histidine.

### <Substitution>

In the present specification, "substitution" refers not only to cases where amino acid residue substitutions are artificially introduced, but also to cases where amino acid residue substitutions are naturally introduced, i.e., cases where the amino acid residues were originally different. In the present specification, the substitution of amino acid residues may be either artificial or natural, but artificial substitution is preferred.

In the present specification, mutations due to amino acid substitution are expressed as a combination of one letter representing the amino acid residue before substitution, a number representing the position of the amino acid residue where the amino acid substitution occurs, and one letter representing the amino acid residue after substitution. For example, if alanine at position 328 is substituted with glycine, it is expressed as "A328G". In addition, the symbol "/" is used to express a combination (concomitance) of two mutations. For example, the combination of alanine at position 328 substituted with glycine and alanine at position 221 substituted with phenylalanine is expressed as "A328G/A221F".

### <Modified esterase>

In the present specification, the term "modified esterase" refers to an esterase obtained by modifying a reference esterase (hereinafter sometimes referred to as "reference esterase"). In the present specification, the reference esterase is typically an esterase having the amino acid sequence of SEQ ID NO: 1 or an esterase having the amino acid sequence of SEQ ID NO: 3. The esterase having the amino acid sequence of SEQ ID NO: 3 is a wild-type esterase derived from Arthrobacter globiformis. The esterase having the amino acid sequence of SEQ ID NO: 1 is a quintuple mutant esterase in which five amino acids (A221F/N222D/F298L/D326V/A328G) are substituted in the wild-type esterase derived from Arthrobacter globiformis.

### <Methyl cyhalothrate>

In the present specification, methyl cyhalothrate (sometimes referred to as "MC") is a substance represented by the substance name methyl 3-(2-chloro-3,3,3-trifluoroprop-1-en-1-yl)-2,2-dimethylcyclopropane-1-carboxylate (structural formula below), and unless otherwise specified, refers to the racemates (1R,3R)- and (1S,3S)-forms.

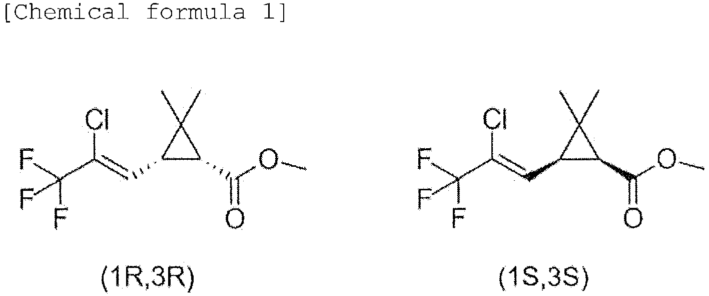

Cyhalothrin acid (sometimes referred to as "CA") is a substance represented by the substance name 3-(2-chloro-3,3,3-trifluoroprop-1-en-1-yl)-2,2-dimethylcyclopropane-1-carboxylic acid (see below [Chemical formula 2]), and unless otherwise specified, refers to a mixture of four isomers: (1R,3R)-, (1R,3S)-, (1S,3R)-, and (1S,3S)-forms.

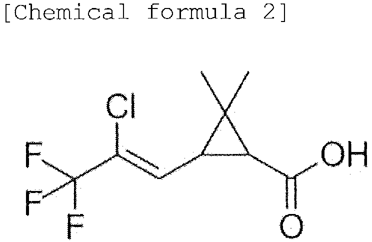

Furthermore, λ-cyhalothric acid (sometimes referred to as "λ-CA" or "(1R,3R)-CA") refers to a substance represented by the substance name (1R,3R)-3-[(1Z)-2-chloro-3,3,3-trifluoroprop-1-en-1-yl]-2,2-dimethylcyclopropane-1-carboxylic acid (see below [Chemical formula 3]).

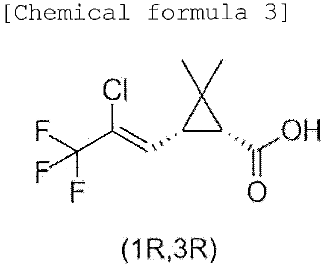

### <Nucleic acid>

In the present specification, "nucleic acid" includes both deoxyribonucleic acid and ribonucleic acid. In one embodiment, the "nucleic acid" may be deoxyribonucleic acid. In another embodiment, the "nucleic acid" may be ribonucleic acid. In another embodiment, the "nucleic acid" may be a chimera of deoxyribonucleic acid and ribonucleic acid.

### 1. Modified esterase

The modified esterase of the present invention is a novel esterase which acts specifically on a substrate having a specific structure (e.g., a compound composed of a dimethylcyclopropane skeleton and a carboxylic acid ester, preferably a compound composed of a dimethylcyclopropane skeleton and prop-1-en, a carboxylic acid ester, particularly preferably (1R,3R)-MC), and as a result, is capable of efficiently synthesizing λ-cyhalothric acid (i.e., (1R,3R)-CA). The substrate is described in more detail in "7. Method for producing (1R,3R)-CA".

The modified esterase of the present invention is an esterase in which a specific amino acid(s) has been substituted in the reference esterase, and specifically, the modified esterase contains or consists of the amino acid sequence specified in (i) to (iii) below:
(i) A modified esterase comprising an amino acid sequence in which at least one amino acid at a site selected from the group consisting of (1) to (24) below in the amino acid sequence of SEQ ID NO: 1 or 3 is substituted with another amino acid;
   (1) V64
   (2) S65
   (3) A102
   (4) A109
   (5) L151
   (6) I153
   (7) Y173
   (8) Y197
   (9) L200
   (10) I211
   (11) F217
   (12) S220
   (13) D222
   (14) D227
   (15) L229
   (16) L242
   (17) A245
   (18) V257
   (19) L298
   (20) T301
   (21) V326
   (22) S329
   (23) A350
   (24) T371
(ii) A modified esterase in which one or more amino acids are further substituted (except for the amino acid site substituted in (i)), added, inserted, or deleted (except for the amino acid site substituted in (i)) in the modified esterase of (i), and the reactivity to (1R,3R)-methyl 3-(2-chloro-3,3,3-trifluoroprop-1-en-1-yl)-2,2-dimethylcyclopropane-1-carboxylate is improved compared to the reactivity of an esterase consisting of the amino acid sequence of SEQ ID NO: 1 or 3;
(iii) A modified esterase in which an amino acid(s) other than the amino acid(s) substituted in (i) is further substituted with another amino acid(s) in the modified esterase of (i), wherein the modified esterase has 70% or more identity with the modified esterase of (i) and has improved reactivity to (1R,3R)-methyl 3-(2-chloro-3,3,3-trifluoroprop-1-en-1-yl)-2,2-dimethylcyclopropane-1-carboxylate compared to the reactivity of the esterase consisting of the amino acid sequence of SEQ ID NO: 1 or 3.

First, the modified esterase of (i) will be explained.

The modified esterase (i) comprises an amino acid sequence in which the original amino acid(s) in the reference esterase is substituted with another amino acid(s) at at least one site of the above (1) to (24). In the esterase of the present invention, the number of substituted amino acids may be usually 1 or more, preferably 2 or more, and more preferably 3 or more (e.g., 3, 4, 5, 6, 7, 8, 9, 10, 11, 12, 13, 14, 15, 16, 17, 18, 19, 20, 21, 22, 23, or 24, or 24 or more).

Regarding the substitution of an original amino acid with another amino acid, the type of the amino acid after substitution is not particularly limited as long as the desired effect of the present invention is achieved, but conservative substitution may be preferable. Conservative substitution of amino acids is a technique well known to those skilled in the art. As an example, the type of amino acid after substitution can be selected with reference to the description of <Types of amino acids> above, but is not limited thereto.

In one embodiment, the number of amino acids to be substituted is one, and the substitution site may be as follows:
(5) L151
(9) L200
(12) S220
(14) D227
(15) L229
(16) L242
(17) A245
(22) S329

In another embodiment, the number of amino acids to be substituted is two, and the substitution site may be as follows:
(5) L151 and (9) L200
(5) L151 and (10) I211
(5) L151 and (13) D222
(5) L151 and (15) L229
(5) L151 and (16) L242
(5) L151 and (22) S329

In another embodiment, the number of amino acids to be substituted is three, and the substitution site may be as follows:
(5) L151, (16) L242, and (3) A102
(5) L151, (16) L242, and (4) A109
(5) L151, (16) L242, and (6) I153
(5) L151, (16) L242, and (7) Y173
(5) L151, (16) L242, and (8) Y197
(5) L151, (16) L242, and (9) L200
(5) L151, (16) L242, and (11) F217
(5) L151, (16) L242, and (13) D222
(5) L151, (16) L242, and (14) D227
(5) L151, (16) L242, and (-) L231
(5) L151, (16) L242, and (18) V257
(5) L151, (16) L242, and (19) L298
(5) L151, (16) L242, and (21) V326
(5) L151, (16) L242, and (22) S329
(5) L151, (16) L242, and (23) A350
(5) L151, (16) L242, and (24) T371

In another embodiment, the number of amino acids to be substituted is four, and the substitution site may be as follows:
(5) L151, (16) L242, (10) I211, and (20) T301
(5) L151, (16) L242, (1) V64, and (2) S65

In another embodiment, the number of amino acids to be substituted is 1 to 4, and the substitution sites may be 1 to 4 selected from the group consisting of the following:
(5) L151, (16) L242, (22) S329, and (9) L200

In a preferred embodiment, the amino acids after substitution at each site may be as follows:
(1) V64M
(2) S65A
(3) A102S
(4) A109T
(5) L151F, L151M, or L151W
(6) I153V
(7) Y173F
(8) Y197F
(9) L200F
(10) I211V or I211M
(11) F217K
(12) S220W
(13) D222G
(14) D227C or D227E
(15) L229G
(16) L242C, L242I, L242M, or L242W
(17) A245T
(18) V257I
(19) L298H
(20) T301A
(21) V326I
(22) S329D, S329G, or S329N
(23) A350D
(24) T371A

In one embodiment, the number of amino acids to be substituted is 1 to 4, and the substitution sites may be 1 to 4 selected from the group consisting of the following:
(5) L151F, (16) L242M, (22) S329N, and (9) L200F

In a preferred embodiment, the modified esterase of the present invention comprises or consists of an amino acid sequence having the following amino acid substitutions in SEQ ID NO: 1 or 3.

### <Modified esterase having one amino acid substitution in the reference esterase>

(E-1) L151F
(E-2) L151M
(E-3) L151W
(E-4) S220W
(E-5) D227C
(E-6) L229G
(E-7) A245T
(E-43) L200F
(E-44) L242M
(E-45) S329N

### <Modified esterase having two amino acid substitutions in the reference esterase>

(E-8) L151F/I211V
(E-9) L151F/I211M
(E-10) L151F/D222G
(E-11) L151F/L229V
(E-12) L151F/L242C
(E-13) L151F/L242I
(E-14) L151F/L242M
(E-15) L151F/L242W
(E-16) L151F/S329D
(E-17) L151F/S329N
(E-46) L151F/L200F

### <Modified esterase having three amino acid substitutions in the reference esterase>

(E-18) L151F/L242M/A102S
(E-19) L151F/L242M/A109T
(E-20) L151F/L242M/I153V
(E-21) L151F/L242M/Y173F
(E-22) L151F/L242M/Y197F
(E-23) L151F/L242M/L200F
(E-24) L151F/L242M/F217K
(E-25) L151F/L242M/D222G
(E-26) L151F/L242M/D227E
(E-27) L151F/L242M/L231M
(E-28) L151F/L242M/V257I
(E-29) L151F/L242M/L298H
(E-30) L151F/L242M/V326I
(E-31) L151F/L242M/S329G
(E-32) L151F/L242M/S329D
(E-33) L151F/L242M/S329N
(E-34) L151F/L242M/A350D
(E-35) L151F/L242M/T371A

### <Modified esterase having four amino acid substitutions in the reference esterase>

(E-36) L151F/L242M/I211V/T301A
(E-37) L151F/L242M/V64M/S65A

In one embodiment, the modified esterase of the present invention may be a fusion of the esterase portion with another polypeptide or protein. In the modified esterase of the present invention, the polypeptide or protein to be fused is not particularly limited, but examples include sequences that are useful for purification, such as multiple histidine residues, and polypeptides that can increase the stability of the esterase during recombinant production.

Next, the modified esterase of (ii) will be explained.

The modified esterase (ii) is the modified esterase (i) described above, to which an additional amino acid modification(s) has been introduced. However, the site(s) of the additional modification(s) is introduced at a site(s) other than the amino acid substitution site(s) specified in the modified esterase (i). The additional amino acid modification(s) may be one of substitution, addition, insertion, and deletion (e.g., only substitution) or two or more of them (e.g., substitution and insertion). In the modified esterase of (ii), the number of amino acid modifications introduced is not particularly limited as long as the desired effect of the present invention is achieved, but is usually 1 or more, for example, 1 to 80, preferably 1 to 70, 1 to 60, 1 to 50, 1 to 40, or 1 to 30, more preferably 1 to 20, 1 to 10, 1 to 8, 1 to 7, 1 to 6, 1 to 5, or 1 to 4, even more preferably 1 to 3, and particularly preferably 1 or 2.

In addition, without wishing to be bound by theory, in the esterase of SEQ ID NO: 1 or 3, the 59th position (serine residue), the 62nd position (lysine residue), and the 148th position (tyrosine residue) are considered to be active catalytic residues. Therefore, it may be preferable not to substitute or delete these sites.

The modified esterase (ii) has the above-mentioned amino acid sequence and is characterized in that its reactivity to (1R,3R)-MC is improved compared to the reactivity of the reference esterase (i.e., the esterase consisting of the amino acid sequence of SEQ ID NO: 1 or 3). More specifically, in a reaction to produce (1R,3R)-CA using (1R,3R)-MC as a substrate, the modified esterase (ii) has an esterase activity that is 1.03 times or more, preferably 1.05 times or more, 1.1 times or more, 1.2 times or more, more preferably 1.5 times or more, and even more preferably 2 times or more, higher than that of the reference esterase (i.e., the esterase having the amino acid sequence of SEQ ID NO: 1 or 3). Alternatively, the modified esterase (ii) has an activity equivalent to that of the modified esterase (i) in a reaction to produce (1R,3R)-CA using (1R,3R)-MC as a substrate.

Next, the modified esterase (iii) will be explained.

The modified esterase (iii) is the modified esterase (i) described above to which an amino acid(s) substitution has been further introduced. However, the site(s) of the additional amino acid substitution(s) is introduced at a site other than the amino acid substitution site specified in the modified esterase (i).

The sequence identity between the modified esterase (iii) and the modified esterase (i) is not particularly limited as long as the desired effect is achieved, but is usually 70% or more, preferably 75% or more, 80% or more, or 85% or more, more preferably 90% or more, 95% or more, 96% or more, 97% or more, or 98% or more, and particularly preferably 99% or more.

Sequence identity can be determined by a method known per se. For example, it can be obtained by the bl2seq program (Tatiana A. Tatsusova, Thomas L. Madden, FEMS Microbiol. Lett., Vol. 174, p. 247-250, 1999) of BLASTPACKAGE [sgi32 bit edition, Version 2.0.12; available from National Center for Biotechnology Information (NCBI)]. When using this method, the parameters can be set as follows: Gap insertion Cost value: 11, Gap extension Cost value: 1.

The modified esterase (iii), like the modified esterase (ii), is characterized in that its reactivity to (1R,3R)-MC is improved compared to the reactivity of the reference esterase. That is, in the reaction of producing (1R,3R)-CA using (1R,3R)-MC as a substrate, the modified esterase (iii) has an esterase activity that is 1.03 times or more, preferably 1.05 times or more, 1.1 times or more, 1.2 times or more, more preferably 1.5 times or more, and even more preferably 2 times or more, higher than that of the reference esterase. Alternatively, the modified esterase (iii) has an activity equivalent to that of the modified esterase (i) in the reaction of producing (1R,3R)-CA using (1R,3R)-MC as a substrate.

### 2. Nucleic acid encoding modified esterase

The present invention also provides a nucleic acid encoding the modified esterase of the present invention (hereinafter, sometimes referred to as the "nucleic acid of the present invention").

The nucleic acid of the present invention is a nucleic acid encoding the modified esterase of the present invention. The nucleic acid of the present invention is not particularly limited as long as it is a nucleic acid encoding the modified esterase of (i) to (iii) described above.

The nucleic acid of the present invention can be prepared by a method known per se. For example, the base sequence shown in SEQ ID NO: 13 is a base sequence that codes for the reference esterase consisting of the amino acid sequence shown in SEQ ID NO: 1, and the base sequence shown in SEQ ID NO: 15 is a base sequence that codes for the reference esterase consisting of the amino acid sequence shown in SEQ ID NO: 3. Therefore, the nucleic acid of the present invention can be appropriately designed by a method known per se, using SEQ ID NO: 13 or 15 as a reference sequence. Note that codon degeneracy may be taken into consideration depending on the host in which the nucleic acid of the present invention is expressed.

In one embodiment, examples of the nucleic acid of the present invention are shown in SEQ ID NOs: 17 to 24. Note that these are nucleic acid sequences that code for modified esterases created based on the reference esterase of SEQ ID NO: 1.
SEQ ID NO:17: L151F mutant
SEQ ID NO:18: L242M mutant
SEQ ID NO:19: S329N mutant
SEQ ID NO:20: L200F mutant
SEQ ID NO:21: L151F/L242M mutant
SEQ ID NO:22: L151F/L242M/S329N mutant
SEQ ID NO:23: L151F/L242M/L200F mutant
SEQ ID NO:24: L151F/L242M/L200F/S329N mutant

The nucleic acid of the present invention is not limited to the nucleic acid sequence itself that encodes the modified esterase of the present invention, and also includes nucleic acid sequences having a homology of 75% or more, preferably 80% or more, 85% or more, 90% or more, more preferably 95% or more, 96%, 97% or more, 98% or more, and particularly preferably 99% or more with the nucleic acid sequence, as long as they encode a polypeptide having an esterase activity equivalent to that of the modified esterase of the present invention.

Here, the "homology" of nucleic acid sequences is calculated using publicly available or commercially available software with an algorithm for comparing a reference sequence with a query sequence. Specifically, BLAST, FASTA, or GENETYX (Software Development Co., Ltd.) can be used. When determining sequence homology using these, the homology score may be determined using default parameters, or the homology score may be determined using appropriately modified parameters.

In addition, nucleic acids that hybridize under stringent conditions with a nucleic acid having a nucleic acid sequence complementary to the nucleic acid of the present invention are also included in the nucleic acid of the present invention, so long as they encode a polypeptide having an enzyme activity equivalent to that of the modified esterase of the present invention. Here, "under stringent conditions" refers to conditions such as 4 hours to overnight incubation at 50°C to 65°C in 0.5% SDS, 5x Denhartz's [0.1% bovine serum albumin (BSA), 0.1% polyvinylpyrrolidone, 0.1% Ficol 400] and 6x SSC containing 100 µg/ml salmon sperm DNA (1x SSC is 0.15M NaCl and 0.015M sodium citrate, pH 7.0). Hybridization under stringent conditions is specifically performed by the following method. That is, a nylon membrane with a DNA library or cDNA library immobilized is prepared, and the nylon membrane is blocked in a prehybridization solution containing 6xSSC, 0.5% SDS, 5xDenhartz's, and 100 µg/ml salmon sperm DNA at 65°C. Then, each probe labeled with 32P is added and incubated at 65°C overnight. The nylon membrane is washed in 6xSSC at room temperature for 10 minutes, in 2xSSC containing 0.1% SDS at room temperature for 10 minutes, and in 0.2xSSC containing 0.1% SDS at 45°C for 30 minutes, and then autoradiography is performed to detect DNA that has specifically hybridized with the probe.

In one embodiment, as described above, the modified esterase of the present invention may be a fusion of the esterase portion with another polypeptide or protein. Thus, the nucleic acid of the present invention may be a base sequence that codes for the esterase portion with another polypeptide or protein.

The method for expressing the nucleic acid of the present invention in a host is not particularly limited, and any method known per se may be used.

### 3. Expression cassette or recombinant vector

The present invention also provides an expression cassette or recombinant vector containing the nucleic acid of the present invention (hereinafter, sometimes referred to as the "cassette of the present invention", the "recombinant vector of the present invention", or the "cassette or recombinant vector of the present invention").

The expression cassette or recombinant vector of the present invention can be prepared by linking a promoter and a terminator to the nucleic acid of the present invention, or by inserting the expression cassette or the nucleic acid of the present invention into an expression vector.

The expression cassette or recombinant vector of the present invention may contain, as control factors, transcription elements such as an enhancer, a CCAAT box, a TATA box, and an SPI site, as necessary, in addition to a promoter and a terminator. These control factors may be operably linked to the nucleic acid of the present invention. Operatively linked means that various control factors that regulate the nucleic acid of the present invention are linked to the nucleic acid of the present invention in a state in which they can operate in a host cell.

In the recombinant vector of the present invention, the vector is preferably an expression vector. As the expression vector, a vector constructed for gene recombination from a phage, a plasmid, or a virus capable of autonomously replicating in a host can be suitably used. Such expression vectors are well known, and examples of commercially available expression vectors include pQE series vectors (Qiagen Co., Ltd.), pDR540, pRIT2T (GE Healthcare Biosciences), pET vectors (Merck). The expression vector can be used in an appropriate combination with the host cell. For example, when E. coli is used as the host cell, examples of suitable combinations include a combination of a pQE vector and a DH5α E. coli strain, a combination of a pET vector and a BL21 (DE3) E. coli strain, or a combination of a pDR540 vector and a JM109 E. coli strain.

### 4. Transformant

The present invention also provides a transformant transformed with the expression cassette or recombinant vector of the present invention (hereinafter, sometimes referred to as "transformant of the present invention").

The host used to produce the transformant of the present invention is not particularly limited as long as it has the following characteristics (1) to (4):
(1) an expression cassette or recombinant vector can be introduced,
(2) the expression cassette or recombinant vector is stable,
(3) it is capable of autonomous replication, and
(4) it is capable of expressing the traits of the gene in the introduced expression cassette or recombinant vector. Examples of such hosts include bacteria belonging to Escherichia genus such as Escherichia coli, Bacillus genus such as Bacillus subtilis, and Pseudomonas such as Pseudomonas putida; filamentous fungi, yeasts, and the like. Also, it may be an animal cell, an insect cell, or a plant.

The transformant of the present invention can be prepared by introducing the nucleic acid of the present invention, the expression cassette of the present invention, or the recombinant vector of the present invention into a host. The site of introduction of the nucleic acid of the present invention and the like is not particularly limited as long as the target gene can be expressed, and may be on a plasmid or on a genome. Specific methods for introducing the expression cassette of the present invention or the recombinant vector of the present invention include, for example, the recombinant vector method and the genome editing method. The conditions for introducing the expression cassette or the recombinant vector into a host may be appropriately set according to the type of the host, etc. When the host is a bacterium, for example, examples of the methods including a method using calcium ion-treated competent cells, and the electroporation method. When the host is a yeast, examples of the methods including the electroporation method, the spheroblast method, and the lithium acetate method. When the host is an animal cell, examples of the methods of the electroporation method, the calcium phosphate method, and the lipofection method. When the host is an insect cell, examples of the methods of the electroporation method, the calcium phosphate method, and the lipofection method. When the host is a plant cell, examples of the methods of the electroporation method, the agrobacterium method, the particle gun method, and the PEG method.

Whether or not the expression cassette of the present invention or the recombinant vector of the present invention has been incorporated into the host can be confirmed by a method known per se, such as PCR, Southern hybridization, or Northern hybridization.

When confirming whether the expression cassette or recombinant vector of the present invention has been incorporated into the host by PCR, for example, the genomic DNA or the expression cassette or recombinant vector may be isolated and purified from the transformant. When the host is, for example, a bacterium, the isolation and purification of the expression cassette or recombinant vector may be carried out based on the lysate obtained by lysing the bacterium. For example, the lysis may be carried out by treating the bacterium with a lytic enzyme such as lysozyme, and, if necessary, protease and other enzymes as well as a surfactant such as sodium lauryl sulfate (SDS) may be used in combination.

Furthermore, physical disruption methods such as freeze-thaw and French press treatment may be combined. Isolation and purification of nucleic acids from the lysate can be performed, for example, by an appropriate combination of deproteinization treatment using phenol treatment and protease treatment, ribonuclease treatment, alcohol precipitation treatment, and commercially available kits.

Cleavage of nucleic acids can be performed according to standard methods, for example, using restriction enzyme treatment. For example, type II restriction enzymes that act on specific nucleotide sequences are used as restriction enzymes. Linkage of nucleic acids to expression cassettes or expression vectors is performed, for example, using DNA ligase.

Then, using the isolated and purified DNA as a template, a primer specific to the DNA of the present invention is designed and PCR is performed. The PCR-obtained amplification product is subjected to agarose gel electrophoresis, polyacrylamide gel electrophoresis, capillary electrophoresis, etc., and stained with ethidium bromide and SYBR Green solution, etc., and the amplification product is detected as a band, so that transformation can be confirmed.

PCR can also be performed using a primer previously labeled with a fluorescent dye, etc., to detect the amplification product. Furthermore, a method of binding the amplification product to a solid phase such as a microplate and confirming the amplification product by fluorescence and enzyme reactions, etc., can also be adopted.

### 5. Method for producing modified esterase

The present invention also provides a method for producing modified esterase, which includes a step of culturing the transformant of the present invention (hereinafter sometimes referred to as the "production method of the present invention").

The culture conditions used in the production method of the present invention may be appropriately set taking into consideration the nutritional and physiological properties of the transformant used.

The transformant can be cultured in a solid or liquid medium, preferably in a liquid medium. For industrial production, aeration and agitation culture is preferred. As the nutrient source for the medium, any substance required for the growth of the transformant may be used. As the carbon source, any assimilable carbon compound may be used, such as glucose, sucrose, lactose, maltose, molasses, pyruvic acid, etc. As the nitrogen source, any assimilable nitrogen compound may be used, such as peptone, meat extract, yeast extract, casein hydrolysate, and soybean meal alkali extract. In addition to the carbon and nitrogen sources, for example, salts such as phosphates, carbonates, sulfates, magnesium, calcium, potassium, iron, manganese, and zinc, specific amino acids, and specific vitamins may be used as necessary.

The culture temperature may be set appropriately within a range in which the transformant of the present invention can grow and the transformant produces modified esterase. In one embodiment, the culture temperature is about 15 to 37°C, but is not limited to this. The culture may be terminated when the modified esterase reaches its maximum yield. The culture time may be, for example, about 12 to 48 hours, but is not limited to this.

The modified esterase expressed using the transformant is present inside the transformant or in the culture medium. The modified esterase may be recovered by a method known per se, but an example of the recovery method is briefly described below.

In one embodiment, when the expressed modified esterase is present inside the transformant, the transformant is separated from the culture supernatant using a method known per se, such as centrifugation. The culture supernatant is removed, and the separated transformant is recovered. The separated transformant is treated using a mechanical method, such as ultrasonication or French pressing, or an enzymatic method, such as lysozyme, and, if necessary, solubilized using an enzyme, such as protease, or a surfactant, such as sodium lauryl sulfate (SDS), to obtain a water-soluble fraction containing the modified esterase.

In another embodiment, when the expressed modified esterase is present in the culture solution, it may be subjected to purification treatment as it is, or the modified esterase in the water-soluble fraction may be concentrated and then subjected to purification treatment. Concentration can be performed, for example, by vacuum concentration, membrane concentration, salting out treatment, fractional precipitation using a hydrophilic organic solvent (e.g., methanol, ethanol, and acetone), etc. In addition, purification of the modified esterase can be performed by appropriately combining methods such as gel filtration, adsorption chromatography, ion exchange chromatography, and affinity chromatography. The modified esterase thus purified can be powdered by freeze drying, vacuum drying, spray drying, etc., as necessary, and distributed on the market.

### 6. Enzyme preparation containing modified esterase

The present invention also provides an enzyme preparation containing the modified esterase of the present invention (hereinafter, sometimes referred to as "the enzyme preparation of the present invention").

The amount of the modified esterase of the present invention contained in the enzyme preparation of the present invention is usually 0.001 to 100% by weight, preferably 0.01 to 100% by weight, 0.1 to 100% by weight, or 1 to 100% by weight, and more preferably 5 to 100% by weight, 10 to 100% by weight, 20 to 100% by weight, 30 to 100% by weight, 40 to 100% by weight, or 50 to 100% by weight, but is not limited thereto.

The enzyme preparation of the present invention may contain only the modified esterase of the present invention, but may also contain other components as long as the desired effect is achieved. Examples of other components include enzymes other than the modified esterase of the present invention, additives, culture residues generated in the production method of the present invention, and the like.

Other enzymes include, for example, amylase (α-amylase, β-amylase, glucoamylase), glucosidase (a-glucosidase, β-glucosidase), galactosidase (α-galactosidase, β-galactosidase), protease (acid protease, neutral protease, alkaline protease), peptidase (leucine peptidase, aminopeptidase), lipase, esterase, cellulase, phosphatase (acid phosphatase, alkaline phosphatase), nuclease, deaminase, oxidase, dehydrogenase, glutaminase, pectinase, catalase, dextranase, transglutaminase, protein deamidase, pullulanase, etc. These other enzymes may be contained alone or in combination of multiple types.

Additives include excipients, buffers, suspending agents, stabilizers, preservatives, and physiological saline. Excipients include starch, dextrin, maltose, trehalose, lactose, D-glucose, sorbitol, D-mannitol, sucrose, and glycerol. Buffers include phosphates, citrates, and acetates. Stabilizers include propylene glycol. Examples of preservatives include phenol, benzalkonium chloride, benzyl alcohol, chlorobutanol, and methylparaben. Examples of preservatives include ethanol, benzalkonium chloride, paraoxybenzoic acid, and chlorobutanol. These additives may be contained alone or in combination.

Examples of culture residues include components derived from the medium, impurity proteins, and bacterial components.

The form of the enzyme preparation of the present invention is not particularly limited, and examples include liquid and solid forms (powder, granules, etc.). The form of the enzyme preparation may be formed by a method known per se.

The modified esterase of the present invention, which is the active ingredient of the enzyme preparation of the present invention, has high enzyme activity in the conversion of (1R,3R)-MC to (1R,3R)-CA. Therefore, in one embodiment of the enzyme preparation of the present invention, the enzyme preparation of the present invention is an enzyme preparation for producing (1R,3R)-CA.

A composition containing (1R,3R)-CA can be efficiently produced by allowing the enzyme preparation of the present invention to act on a composition containing a compound composed of a dimethylcyclopropane skeleton and a carboxylic acid ester (e.g., (1R,3R)-MC) under appropriate conditions. The substrate of the enzyme preparation of the present invention will be described in detail in the "Method for producing (1R,3R)-CA" below.

### 7. Method for producing (1R,3R)-CA

The present invention also provides a method for producing (1R,3R)-3-[(1Z)-2-chloro-3,3,3-trifluoroprop-1-en-1-yl]-2,2-dimethylcyclopropane-1-carboxylic acid, which comprises the step of acting the modified esterase of the present invention on (1R,3R)-methyl 3-(2-chloro-3,3,3-trifluoroprop-1-en-1-yl)-2,2-dimethylcyclopropane-1-carboxylate (i.e., (1R,3R)-MC) (hereinafter, sometimes referred to as the "method for producing (1R,3R)-CA of the present invention").

In the method for producing (1R,3R)-CA of the present invention, the substrate, (1R,3R)-MC, may be purified, or may be a composition containing other components (hereinafter, sometimes referred to as "(1R,3R)-MC-containing composition"). The (1R,3R)-MC-containing composition may be a highly pure (1R,3R)-MC (e.g., purity of 98% or more), or a substrate in which (1R,3R)-MC and (1S,3S)-MC coexist (e.g., a mixed MC with (1R,3R) : (13,33)=1:1).

The (1R,3R)-MC content in the (1R,3R)-MC-containing composition is not particularly limited, but may be, for example, 20% or more. From the viewpoint of further increasing the (1R,3R)-CA content in the product, the (1R,3R)-MC content in the substrate is preferably 30% or more, more preferably 40% or more, and even more preferably 45% or more. The upper limit of the content range is not particularly limited, but may be, for example, 100% or less, preferably 90% or less, and more preferably 70% or less. The origin of the (1R,3R)-MC-containing composition is not particularly limited, and may be, for example, a chemically synthesized composition.

As long as (1R,3R)-CA can be produced, there are no particular limitations on the reaction time, reaction temperature, pH of the reaction solution, etc. when the modified esterase is allowed to act on the substrate (e.g., the (1R,3R)-MC-containing composition). The reaction time is, for example, 10 minutes to 96 hours, preferably 1 hour to 72 hours, and more preferably 12 hours to 48 hours. The reaction temperature is, for example, 10 to 90°C, preferably 20 to 80°C, and more preferably 30 to 50°C. The pH of the reaction solution is, for example, 3 to 12, preferably 6 to 11, and more preferably 8 to 11. These reaction conditions are appropriately selected depending on the raw materials used and the desired product. The optimal reaction conditions can be determined through preliminary experiments.

By using the method for producing (1R,3R)-CA of the present invention, a (1R,3R)-CA-containing composition can be produced very efficiently. One embodiment of the method for producing a (1R,3R)-CA-containing composition of the present invention includes the following steps (1) and (2). Note that after step (2), a step of recovering (1R,3R)-CA from the obtained (1R,3R)-CA-containing composition may be added.
(1) Step of providing a (1R,3R)-MC-containing composition
(2) Step of treating the provided (1R,3R)-MC-containing composition with the modified esterase of the present invention

The content of (1R,3R)-CA in the (1R,3R)-CA-containing composition obtained by the method for producing (1R,3R)-CA of the present invention is not particularly limited, but is usually 1% by weight or more, preferably 2% by weight or more, 3% by weight or more, 4% by weight or more, or 5% by weight or more, more preferably 10% by weight or more, 12% by weight or more, or 14% by weight or more, and particularly preferably 16% by weight or more.

The (1R,3R)-CA-containing composition is preferably used as an intermediate for agricultural chemicals.

Although not wishing to be bound by theory, the substrate of the modified esterase of the present invention may be a compound represented by the following formula I, which is composed of a dimethylcyclopropane skeleton and a carboxylic acid ester.

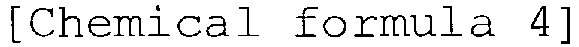

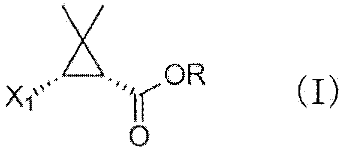

In formula I, the structures of X₁ and R are not particularly limited and may be any structure. Examples of X₁ include, but are not limited to, halogeno groups (fluoro, chloro, bromo, iodo, aster, etc.), alkyl groups (methyl, ethyl, propyl, isopropyl, n-butyl, isobutyl, sec-butyl, tert-butyl, etc.), and aryl groups (phenyl, biphenyl, naphthyl, etc.). Examples of R include, but are not limited to, alkyl groups (methyl, ethyl, propyl, isopropyl, n-butyl, isobutyl, sec-butyl, tert-butyl, etc.) and aryl groups (phenyl, biphenyl, naphthyl, etc.). The carbon chains of these substituents may be linear or branched, and may have a cyclic structure or an unsaturated bond in the middle or at the end.

These substituents may be further substituted with at least one of the following optional substituents.
(1) halogen group,
(2) hydroxyl group,
(3) cyano group,
(4) nitro group,
(5) carboxyl group,
(6) alkenyl group (C₂₋₁₀ alkenyl group; e.g., vinyl, allyl, propenyl, butenyl, pentenyl, hexenyl, heptenyl, butadienyl, hexatrienyl, and each isomer thereof),
(7) alkynyl groups (C₂₋₁₀ alkynyl groups; e.g., ethynyl, propynyl, butynyl, pentynyl, hexynyl, and each isomer thereof),
(8) halogenoalkyl groups (e.g., monofluoromethyl, difluoromethyl, trifluoromethyl, monofluoroethyl, difluoroethyl, trifluoroethyl, chloromethyl, chloroethyl, dichloroethyl, and each isomer thereof),
(9) cyclic alkyl groups (which may contain heteroatoms in the ring) (e.g., cyclopropyl, cyclobutyl, cyclopentene, cyclohexyl, tetrahydrofuranyl, tetrahydropyranyl, aziridinyl, azetidinyl, pyrrolidinyl, piperidinyl, morpholinyl),
(10) aryl groups (e.g., phenyl, naphthyl),
(11) heteroaryl groups (e.g., pyridyl, pyridazinyl, pyrimidinyl, pyrazinyl, furyl, thiophenyl, pyrrolyl, pyrazolyl, imidazolyl, triazolyl (e.g., 1,2,3-triazolyl, 1,2,4-triazolyl), tetrazolyl, oxazolyl, isoxazolyl, thiazolyl, isothiazolyl, oxadiazolyl (e.g., 1,2,3-oxadiazolyl, 1,2,4-oxadiazolyl, 1,3,4-oxadiazolyl), thiadiazolyl (e.g., 1,2,3-thiadiazolyl, 1,2,4-thiadiazolyl, 1,3,4-thiadiazolyl), benzofuryl, benzothiophenyl, indolyl, isoindolyl, benzoxazolyl, benzothiazolyl, benzimidazolyl, indazolyl, benzisoxazolyl, benzisothiazolyl, benzoxadiazolyl, benzothiadiazolyl, purinyl, quinolinyl, isoquinolinyl, cinnolinyl, phthalazinyl, quinazolinyl, quinoxalinyl, pteridinyl, imidazooxazolyl, imidazothiazolyl, imidazoimidazolyl), (12) alkoxy groups (e.g., methoxy, ethoxy, n-propoxy, isopropoxy, n-butoxy, isobutoxy, sec-butoxy, tert-butoxy, n-pentyloxy, isopentyloxy, tert-pentyloxy, neopentyloxy, 2-pentyloxy, 3-pentyloxy, n-hexyloxy, 2-hexyloxy),
(13) alkylthio group (e.g., methylthio, ethylthio, n-propylthio, isopropylthio, n-butylthio, isobutylthio, sec-butylthio, tert-butylthio, n-pentylthio, isopentylthio, tert-pentylthio, neopentylthio, 2-pentylthio, 3-pentylthio, n-hexylthio, 2-hexylthio),
(14) alkoxy group (same as (12) above) substituted with aryl group (same as (10) above),
(15) alkylthio group (same as (13) above) substituted with aryl group (same as (10) above),
(16) alkoxy group (same as (12) above) substituted with heteroaryl group (same as (11) above),
(17) alkylthio group (same as (13) above) substituted with heteroaryl group (same as (11) above),
(18) cyclic alkyloxy group (which may contain a hetero atom in the ring) (e.g., cyclopropyloxy, cyclobutyloxy, cyclopentyloxy, cyclohexyloxy, tetrahydrofuranyloxy, tetrahydropyranyloxy, aziridinyloxy, azetidinyloxy, pyrrolizinyloxy, piperidinyloxy, morpholinyloxy),
(19) aryloxy group (e.g., a group in which an aryl group (same as (10) above) is bonded to an oxygen atom),
(20) heteroaryloxy group (e.g., a group in which a heteroaryl group (same as (11) above) is bonded to an oxygen atom),
(21) halogenoalkoxy group (e.g., a group in which a halogenoalkyl group (same as (8) above) is bonded to an oxygen atom),
(22) halogenoalkoxy group (e.g., a group in which a halogenoalkyl group (same as (8) above) is bonded to a sulfur atom),
(23) alkoxy group (same as (12) above) substituted with a hydroxyl group,
(24) alkoxy group (same as (12) above) substituted with an alkoxy group (same as (12) above),
(25) amino group,
(26) amino group mono- or di-substituted with an alkyl group,
   Here, "alkyl group" refers to C₁₋₆ alkyl groups, specifically methyl, ethyl, n-propyl, isopropyl, n-butyl, isobutyl, sec-butyl, tert-butyl, n-pentyl, isopentyl, tert-pentyl, neopentyl, 2-pentyl, 3-pentyl, n-hexyl, 2-hexyl, etc.
(27) carbamoyl group,
(28) carbamoyl group mono- or di-substituted with alkyl group (same as "alkyl group" in (26) above) (e.g., methylcarbamoyl, ethylcarbamoyl, dimethylcarbamoyl, diethylcarbamoyl, ethylmethylcarbamoyl),
(29) sulfamoyl group,
(30) sulfamoyl group mono- or di-substituted with alkyl group (same as "alkyl group" in (26) above) (e.g., methylsulfamoyl, ethylsulfamoyl, dimethylsulfamoyl, diethylsulfamoyl, ethylmethylsulfamoyl),
(31) alkanoyl group (e.g., carbonyl group with hydrogen atoms or alkyl groups (same as "alkyl group" in (26) above) bonded to a carbon atom),
(32) aroyl group (e.g., carbonyl group with aryl group (same as (10) above) bonded to the carbon atom),
(33) alkylsulfonylamino group (e.g., sulfonylamino group substituted with alkyl group (same as "alkyl group" in the above (26)) group),
(34) arylsulfonylamino group (e.g., sulfonylamino group substituted with an aryl group (same as (10) above)),
(35) heteroarylsulfonylamino group (e.g., sulfonylamino group substituted with a heteroaryl group (same as (11) above)),
(36) acylamino group (e.g., amino group substituted with an acyl group),
   Here, the "acyl group" refers to an acyl group having a C₁₋₆ alkyl group or a C₆₋₁₀ aryl group. Here, the "C₁₋₆ alkyl group" refers to the above "alkyl group" having 1 to 6 carbon atoms, and the "C₆₋₁₀ aryl group" refers to the above "aryl group" having 6 to 10 carbon atoms. Specific examples of acyl groups include acetyl group, propionyl group, butyroyl group, isobutyroyl group, valeroyl group, isovaleroyl group, pivaloyl group, hexanoyl group, acryloyl group, methacryloyl group, crotonoyl group, isocrotonoyl group, benzoyl group, naphthoyl group, etc.,
(37) alkoxycarbonylamino group (e.g., carbonylamino group substituted with alkoxy group (same as (12) above)),
(38) alkylsulfonyl group (e.g., sulfonyl group substituted with alkyl group (same as "alkyl group" in (26) above)),
(39) alkylsulfinyl group (e.g., sulfinyl group substituted with alkyl group (same as "alkyl group" in (26) above)),
(40) alkoxycarbonyl group (e.g., methoxycarbonyl group, ethoxycarbonyl group), etc.

When two or more substituents are present, they may be the same or different.

The modified esterase of the present invention can be suitably used not only for the production of the above-mentioned (1R,3R)-CA, but also for the production of the following compound of formula II by acting the modified esterase of the present invention on the compound of formula I.

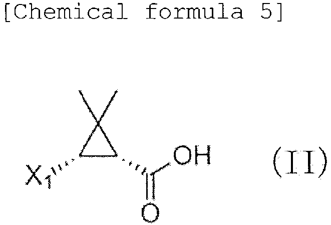

Based on this aspect, the present invention also provides a method for producing a compound of formula II, which includes acting the modified esterase of the present invention on the compound of formula I (hereinafter sometimes referred to as "the method for producing the compound of the present invention").

In the method for producing the compound of the present invention, the conditions under which the modified esterase of the present invention acts on the compound of formula I are not particularly limited, so long as the modified esterase of the present invention hydrolyzes the compound of formula I to produce the compound of formula II. Such conditions may be the same as or different from the reaction conditions under which a known esterase can hydrolyze an ester. Examples include, but are not limited to, the following conditions.

Reaction time: usually 10 minutes to 120 hours, preferably 1 hour to 96 hours, more preferably 12 hours to 72 hours. Reaction temperature: usually 10 to 90°C, preferably 20 to 80°C, more preferably 30 to 50°C.

pH of the reaction solution: usually 3 to 12, preferably 6 to 11, more preferably 7 to 11.

In the method for producing the compound of the present invention, the solvent used when the modified esterase of the present invention acts on the compound of formula I is also not particularly limited as long as it is a solvent in which the modified esterase of the present invention hydrolyzes the compound of formula I to produce the compound of formula II. Examples of such solvents include, but are not limited to, water.

In a preferred embodiment of the method for producing the compound of the present invention, the substrate of the modified esterase of the present invention may be a compound represented by the following formula III.

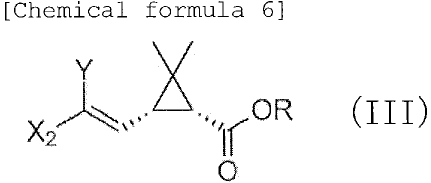

In formula III, the structures of X₂ and Y are not particularly limited and may be any structure. Examples of X₂ and Y include, but are not limited to, hydrogen, halogeno group (fluoro group, chloro group, bromo group, iodo group, astat group, etc.), alkyl group (methyl group, ethyl group, propyl group, isopropyl group, n-butyl group, isobutyl group, sec-butyl group, tert-butyl group, etc.), and aryl group (phenyl group, biphenyl group, naphthyl group, etc.). Furthermore, the carbon chains of these substituents can be either straight or branched, and may have a cyclic structure or an unsaturated bond in the middle or at the end. These substituents may be further substituted with at least one of the above-mentioned (1) to (40) optional substituents. When two or more substituents are present, they may be the same or different. R is as described above.

In a preferred embodiment, X₂ can be a chloro group, a methyl group, or a trifluoromethyl group. Also, Y can be a chloro group or a methyl group, and R can be a methyl group or an ethyl group.

In a more preferred embodiment, X₂ can be a chloro group, a methyl group, or a trifluoromethyl group, and Y can be a chloro group or a methyl group. R can be a methyl group or an ethyl group.

In a further preferred embodiment, X₂ can be a trifluoromethyl group, Y can be a chloro group, and R can be a methyl group (i.e., (1R,3R)-methyl cyhalothrate).

Alternatively, X₂ and Y can be methyl groups, and R can be an ethyl group (i.e., (1R,3S)-ethyl chrysanthemate).

Alternatively, X₂ and Y can be chloro groups and R can be a methyl group (i.e., (1R,3R)-methyl permethrate).

The present invention will be explained in more detail in the following examples, but the present invention is not limited to these examples.

### [Example]

The artificial esterase of SEQ ID NO.: 1, which is a quintuple mutant of the wild-type esterase (SEQ ID NO.: 3) derived from Arthrobacter globiformis and has high reactivity with ethyl chrysanthemate, has slight reactivity with (1R,3R)-MC. Therefore, using this esterase as the reference esterase, the present inventors attempted to create a modified esterase with improved reactivity with (1R,3R)-MC by protein engineering techniques. The detailed procedure is shown below.

### 1. Creation of a mutant library

A mutant library was created using the following method.

### (1) Introduction of mutations and transformation

PCR primers for introducing mutations were designed in which the NNK codon was introduced to the amino acid near the active center, and PCR was performed under the following conditions to introduce mutations.

### <Primers>

The primers used to create the 151st position mutant library and the 242nd position mutant library are listed below.

Primers for constructing mutant library at position 151:
F-primer sequence: NNKACCATCGGCGTCTTCATGGAGGAG (SEQ ID NO.: 25)
R-primer sequence: GGCGTGGTACCCGAAGGCGGTCCC (SEQ ID NO.: 26)
Primers for constructing mutant library at position 242:
F-primer sequence: NNKAGTTCAGCCGCCGGAGTCGCC (SEQ ID NO.: 27)
R-primer sequence: GCCGGCTGCGCGGACCTCGCGGAGG (SEQ ID NO.: 28)

### <Template plasmid>

pET21b (introducing the reference esterase gene sequence) approximately 30 ng/µL

### <PCR reaction composition> (total volume 20 µL)

PrimeSTAR GXL Premix (Takarabio): 10 µL
Template (approximately 30 ng/µL): 1 µL
F-primer: 0.2 µL
R-primer: 0.2 µL
Mix these and adjust to 20 µL with ultrapure water (Milli Q water).

### <PCR conditions>

(1) 94°C, 30 seconds
(2) <96°C, 10 seconds, 60°C, 15 seconds, 68°C, 6 minutes 30 seconds> x 20 cycles
(3) 68°C, 5 minutes
(4) Leave at 4°C

1.5 µL of DpnI was added to 15 µL of PCR reaction solution and treated (37°C, 2 hours or more). Next, 2 µL of DpnI treatment solution was used to perform ligation treatment (16°C, O/N) with the following ligation reaction composition.

### <Ligation reaction composition> (total volume 15 µL)

DpnI treated PCR product: 2 µL
T4 Polynucleotide Kinase: 1 µL
Ligation High (Toyobo): 5 µL
Sterile purified water: 7 µL

2.5 µL of the ligation reaction solution was used to transform 25 µL of E. coli BL21(DE3) (at 42°C for 45 seconds, then rapidly cooled on ice). Approximately 100 µL of the transformation solution was applied to an LB agar medium plate supplemented with ampicillin (final concentration 0.1 mg/mL) and cultured at 37°C O/N (preculture).

### (2) Obtaining enzyme extract

Each mutant strain after preculture was inoculated into 1 mL of TB medium supplemented with ampicillin (final concentration 0.1 mg/mL). They were cultured at 33°C for 48 hours (IPTG (final concentration 0.1 mM) was added 24 hours after the start of culture) and centrifuged (3,000 g × 15 minutes). The supernatant was removed and the cells were collected, after which the cells were lysed with a lysing agent (25°C, 2 hours or more). The lysate was centrifuged (3,000 g × 15 minutes), and the supernatant was collected and used as the enzyme extract.

### (3) Preparation of combinatorial mutants

Using the obtained mutants as a template, PCR was performed in the same manner as in (1) above. The PCR product was collected, and an enzyme extract was obtained in the same manner as in (2) above.

### (4) Preparation of multiple mutants

Using the L151F/L242M mutants as a template, random mutagenesis was performed by PCR using the Diversify PCR Random Mutagenesis Kit (Takarabio). The following primers were used.

Primers for random mutagenesis:
F-primer sequence: ATATGACCATGATTACGCC (SEQ ID NO.: 29)
R-primer sequence: TGGTGCTCGAGCTACTGAG (SEQ ID NO.: 30)

Following the usual method, after ligation treatment, 25 µL of E. coli BL21(DE3) was transformed. As an indicator of improved reactivity, trans-pNP chrysanthemum acid was used, and screening was performed using the amount of released pNP as an indicator. For the selected mutant strains, enzyme extracts were obtained using the same procedure as in (2) above.

### 2. Evaluation of mutants

### 2-1. Evaluation of mutants

The mutants were evaluated using the following method. Substrate: Methyl cyhalothrate (mixture of (1R,3R) form and (1S,3S) form = 50:50, purity 90% or higher)

5 µL of substrate was dispensed into a reaction vessel, 95 µL of enzyme extract (pH7.5) was added, and the reaction mixture was treated at 40°C for 24 hours until the reaction stabilized. After treatment, 400 µL of acetonitrile was added. After thorough stirring, the mixture was centrifuged, and 5 µL of the organic layer was collected and diluted with 200 µL of 80% acetonitrile solution. The diluted solution was analyzed by ultra-high performance liquid chromatography (UPLC). The analytical conditions are as follows.

### <Ultra-high performance liquid chromatography (UPLC) analytical conditions>

Apparatus: ACQUITY UPLC H-Class system PDA system (Waters Corporation)
Column: ACQUITY UPLC BEH C18 1.7 µm (Waters Corporation) Mobile phase: Acetonitrile/water/TFA = 60/40/0.1
Flow rate: 1.0 mL/min
Injection volume: 1 µL
Analysis time: 2 min/sample
PDA detector: 210-400 nm
Resolution: 4.8 nm

Changes in reactivity to (1R,3R)-methyl cyhalothrate were evaluated based on the reactivity of the "reference esterase (consisting of the amino acid sequence of SEQ ID NO.: 1)". Specifically, the value of "amount of cyhalothric acid produced in the reaction using the mutant" / "amount of cyhalothric acid produced in the reaction using the reference esterase" was calculated from the area of the UPLC area, and if this value exceeded 1, it was determined that the reactivity to (1R,3R)-methyl cyhalothrate had improved.

The evaluation results are shown in Table 1. The following mutations were identified as having increased reactivity to (1R,3R)-methyl cyhalothrate. In the reaction using the reference esterase, 10% of (1R,3R)-MA was converted to (1R,3R)-CA (conversion rate for the total substrate was 5%).

**[Table 1]**

| | | Relative activity to reference esterase (%) |
|---|---|---|
| Comparative Example 1 | SEQ ID NO: 1 (reference esterase) | 100 |
| Example 1 | L151F mutant | 192 |
| Example 2 | L151M mutant | 167 |
| Example 3 | L151W mutant | 175 |
| Example 4 | S220W mutant | 121 |
| Example 5 | D227C mutant | 116 |
| Example 6 | L229G mutant | 129 |
| Example 7 | A245T mutant | 136 |
| Example 43 | L200F mutant | 108 |
| Example 44 | L242M mutant | 113 |
| Example 45 | S329N mutant | 165 |

### 2-2. Evaluation of double mutants

7 µL of substrate was dispensed into a reaction vessel, 93 µL of enzyme extract (pH7.5) was added, and the reaction mixture was treated at 40°C for 24 hours until the reaction stabilized. After treatment, 400 µL of acetonitrile was added. After thorough mixing, the mixture was centrifuged, and 5 µL of the organic layer was collected and diluted with 200 µL of 80% acetonitrile solution. The diluted solution was analyzed and evaluated in the same manner as in 2-1 using ultra-high performance liquid chromatography (UPLC).

The evaluation results are shown in Table 2. The following mutations were identified as having increased reactivity to (1R,3R)-methyl cyhalothrate.

**[Table 2]**

| | | Relative activity to reference esterase (%) |
|---|---|---|
| Comparative Example 1 | SEQ ID NO: 1 (reference esterase) | 100 |
| Example 1 | L151F mutant | 192 |
| Example 8 | L151F/I211V mutant | 246 |
| Example 9 | L151F/I211M mutant | 225 |
| Example 10 | L151F/D222G mutant | 263 |
| Example 11 | L151F/L229V mutant | 248 |
| Example 12 | L151F/L242C mutant | 219 |
| Example 13 | L151F/L242I mutant | 228 |
| Example 14 | L151F/L242M mutant | 268 |
| Example 15 | L151F/L242W mutant | 228 |
| Example 16 | L151F/S329D mutant | 204 |
| Example 17 | L151F/S329N mutant | 273 |
| Example 46 | L151F/L200F mutant | 203 |

### 2-3. Evaluation of multiple mutants

15 µL of substrate was dispensed into a reaction vessel, 85 µL of enzyme extract (pH7.5 or pH10) was added, and the reaction mixture was treated at 40°C for 24 hours until the reaction stabilized. After treatment, 400 µL of acetonitrile was added. After thorough mixing, the mixture was centrifuged, and 5 µL of the organic layer was collected and diluted with 200 µL of 80% acetonitrile solution. The diluted solution was analyzed and evaluated by ultra-high performance liquid chromatography (UPLC) in the same manner as 2-1.

The evaluation results are shown in Table 3 (pH7.5) and Table 4 (pH10). The following mutations were identified as those that showed increased reactivity to (1R,3R)-methyl cyhalothrate at pH7.5 and pH10.

**[Table 3]**

| | | Relative activity to reference esterase (%) (pH 7.5) |
|---|---|---|
| Comparative Example 1 | SEQ ID NO: 1 | 100 |
| Example 14 | L151F/L242M mutant | 268 |
| Example 18 | L151F/L242M/A102S mutant | 321 |
| Example 19 | L151F/L242M/A109T mutant | 375 |
| Example 20 | L151F/L242M/1153V mutant | 292 |
| Example 21 | L151F/L242M/Y173F mutant | 294 |
| Example 22 | L151F/L242M/Y197F mutant | 321 |
| Example 23 | L151F/L242M/L200F mutant | 294 |
| Example 24 | L151F/L242M/F217K mutant | 308 |
| Example 25 | L151F/L242M/D222G mutant | 270 |
| Example 26 | L151F/L242M/D227E mutant | 297 |
| Example 27 | L151F/L242M/L231M mutant | 316 |
| Example 28 | L151F/L242M/V257I mutant | 321 |
| Example 29 | L151F/L242M/L298H mutant | 321 |
| Example 30 | L151F/L242M/V326I mutant | 294 |
| Example 31 | L151F/L242M/S329G mutant | 302 |
| Example 32 | L151F/L242M/S329D mutant | 388 |
| Example 33 | L151F/L242M/S329N mutant | 364 |
| Example 34 | L151F/L242M/A350D mutant | 302 |
| Example 35 | L151F/L242M/T371A mutant | 321 |
| Example 36 | L151F/L242M/I211V/T301A mutant | 348 |
| Example 37 | L151F/L242M/V64M/S65A mutant | 321 |

**[Table 4]**

| | | Relative activity to reference esterase (%) (pH10) |
|---|---|---|
| Comparative Example 2 | SEQ ID NO: 1 | 100 |
| Example 38 | L151F/L242M mutant | 245 |
| Example 39 | L151F/L242M/I153V mutant | 301 |
| Example 40 | L151F/L242M/L200F mutant | 318 |
| Example 41 | L151F/L242M/D222G mutant | 314 |
| Example 42 | L151F/L242M/L200F/S329N mutant | 316 |

### 3. Evaluation of stereoselectivity of mutants

Three types of modified esterase (L151F/L242M/S329N, L151F/L242M/L200F, L151F/L242M/L200F/S329N) were prepared as follows. Each mutant strain was inoculated into 1 L of TB medium supplemented with ampicillin (final concentration 0.1 mg/mL). The strains were cultured at 33°C for 48 hours (IPTG (final concentration 0.1 mM) was added 24 hours after the start of culture) and centrifuged (3,000 g × 15 minutes). The supernatant was removed and the cells were collected, after which the cells were lysed with a lysing agent (25°C, 2 hours or more). The lysate was centrifuged (3,000 g × 15 minutes) and the supernatant was collected. The supernatant was concentrated using ultrafiltration and freeze-dried to obtain the enzyme powder.

### 3-1. Method for evaluating stereoselectivity

Three mutant enzyme powder samples (L151F/L242M/S329N, L151F/L242M/L200F, L151F/L242M/L200F/S329N) and the reference esterase were used to evaluate the reactivity to methyl cyhalothrate ((1R,3R):50%, (1S,3S):50%). 7.5 g of methyl cyhalothrate was added to a 100 mL Erlenmeyer flask, and 43.9 mL of 100 mM Gly-NaOH Buffer pH10 was added and stirred for a while. After that, each enzyme sample was added to each final concentration (reference esterase: 44 U/mL, L151F/L242M/S329N: 21 U/mL, L151F/L242M/L200F: 28 U/mL, L151F/L242M/L200F/S329N: 6 U/mL), and the pH was adjusted to about 9.8 with 20% NaOH aq., after which the reaction was carried out at 40°C for up to 48 hours. 400 µL of acetonitrile and 100 µL of the reaction solution were mixed well in a 1.5 mL tube. The mixed sample was centrifuged at 10,000G for 5 minutes, and then 5 µL of the supernatant was mixed with 200 µL of 80% acetonitrile-water to prepare a sample, which was then analyzed by HPLC.

In addition, a solution of (1R,3R)-cyhalothric acid with a known concentration was prepared and analyzed by HPLC using the same procedure to create a calibration curve. Using this calibration curve, the amount of (1R,3R)-cyhalothric acid produced by the enzyme reaction was determined, and the conversion rate was calculated. The ratio of (1R,3S)-cyhalothric acid in the product ((1R,3S)-cyhalothric acid or (1R,3R)-cyhalothric acid) was taken as the optical purity.

The definition and measurement method of esterase activity are as follows:

When esterase is applied to p-Nitrophenyl-butyrate (abbreviation: p-NPB) as a substrate, p-nitrophenol is produced, and the absorbance at a wavelength of 416 nm increases. 4-Nitrophenyl butyrate (p-NPB, SIGMA) was dissolved in 0.1M PIPES buffer (pH 7.0) containing 1% Triton X-100 to obtain a p-NPB solution with a final concentration of 2.5 mM. Using the automatic analyzer TBA-120FR, 15 µL of the measurement sample (enzyme solution) and 300 µL of the p-NPB solution were mixed and the change in absorbance at 416 nm was measured. The amount of p-nitrophenol produced was calculated from the change in absorbance at 416 nm, and the amount of enzyme that produces 1 µmol of p-nitrophenol per minute is defined as 1 unit.

The HPLC conditions are as follows:

### Evaluation of reactivity to methyl cyhalothrate <HPLC method>

Apparatus: Waters H-class system
Column: CHIRALCEL (registered trademark) OD-RH 4.6 mmΦ×150 mmL
Mobile phase: Acetonitrile/water/TFA
Gradient conditions: 30/70/0.1 (30 min) → 45/55/0.1 (5 min) - 45/55/0.1 (10 min) → 30/70/0.1 (3 min) → 30/70/0.1 (5 min)
Flow rate: 1.0 mL/min
Injection volume: 5 µL
Analysis time: 53 min/sample
PDA detector: 210 nm

### Evaluation of reactivity to ethyl chrysanthemate

<HPLC method>
Apparatus: Agilent 1290 HPLC
Column: CHIRALCEL (registered trademark) OJ-RH 4.6 mmΦ×150 mmL Mobile phase: Acetonitrile/water/TFA
Gradient conditions: 30/70/0.1 (30 min) → 45/55/0.1 (5 min) - 45/55/0.1 (10 min) → 30/70/0.1 (5 min) → 30/70/0.1 (5 min) Flow rate: 1.0 mL/min
Injection volume: 5 µL
Analysis time: 55 min/sample
PDA detector: 210 nm

The reactivity evaluation for methyl permethrate was performed in the same manner as for ethyl chrysanthemate.

### 3-2. Evaluation results of stereoselectivity

When the amount of product produced in the reaction catalyzed by the reference esterase from (1R,3R)-methyl cyhalothrate to (1R,3R)-cyhalothric acid was taken as 100%, the relative activity of the mutant enzymes was 235% for L151F/L242M/S329N, 230% for L151F/L242M/L200F, and 220% for L151F/L242M/L200F/S329N.

Of the products, the ratio of (1R,3R)-cyhalothric acid (optical purity) was 96% for the reference esterase, 96% for L151F/L242M/S329N, 95% for L151F/L242M/L200F, and 96% for L151F/L242M/L200F/S329N. Although all mutant enzymes showed higher conversion rates than the reference esterase, there was no effect on stereoselectivity.

### 4. Evaluation of reactivity to similar substrates

### 4-1. Evaluation method

Two mutant enzyme powder samples (L151F/L242M/S329N, L151F/L242M/L200F) and the reference esterase (EYN7) were used to evaluate the reactivity to ethyl chrysanthemate ((1R,3R) :32.5%, (1S,3S):32.5%, (1R,38):17.5%, (1S,3R):17.5%) and methyl permethrate ((1R,3S):32.5%, (1S,3R):32.5%, (1R,3R):17.5%, (1S,3S):17.5%). 7.5 g of substrate (ethyl chrysanthemate or methyl permethrate) was added to a 100 mL Erlenmeyer flask. 41.75 mL of 100 mM Gly-NaOH Buffer pH10 was added and stirred for a while. After that, each enzyme sample was added to the respective final concentration (reference esterase: 70 U/mL, L151F/L242M/S329N: 33 U/mL, L151F/L242M/L200F: 44 U/mL), and the pH was adjusted to around 9.8 with 20% NaOH aq., after which the reaction was carried out at 40°C for up to 48 hours.

400 µL of acetonitrile and 100 µL of the reaction solution were mixed thoroughly in a 1.5 mL tube. The mixed sample was centrifuged at 10,000G for 5 minutes, and then 25 µL of the supernatant was mixed with 1 mL of 80% acetonitrile-water to prepare a sample, which was then analyzed by HPLC.

In addition, a solution of (1R,3S)-chrysanthemic acid with a known concentration was prepared and analyzed by HPLC using the same procedure to create a calibration curve. Using this calibration curve, the amount of (1R,3S)-chrysanthemic acid produced by the enzyme reaction was determined, and the conversion rate was calculated.

(1R,3R)-chrysanthemic acid was evaluated by comparing its peak area value with that of (1R,3S)-chrysanthemic acid. The ratio of (1R,3S)-chrysanthemic acid in the products ((1R,3S)-chrysanthemic acid or (1R,3R)-chrysanthemic acid) was used as the optical purity. The reactivity of methyl permethrate was evaluated by the peak area values of (1R,3R)-permethric acid and (1R,3S)-permethric acid. In the products, the ratio of (1R,3S)-permethric acid was defined as the optical purity. It was evaluated by the peak area values of (1R,3R)-permethric acid and (1R,3S)-permethric acid. The ratio of (1R,3R)-permethric acid in the products ((1R,3R)-permethric acid or (1R,3S)-permethric acid) was defined as the optical purity.

### 4-2. Evaluation results

### Conversion rate to (1R,3S)-chrysanthemic acid and specificity to (1R,3S)-ethyl chrysanthemate

When the amount of product produced in the reaction catalyzed by the reference esterase from (1R,3S)-ethyl chrysanthemate to (1R,3S)-chrysanthemic acid was taken as 100%, the relative activity of the mutant enzymes was 126% for L151F/L242M/S329N and 128% for L151F/L242M/L200F.

The optical purity was 94% for the reference esterase, 100% for L151F/L242M/S329N, and 94% for L151F/L242M/L200F. Although all mutant enzymes showed higher conversion rates than the reference esterase, there was no effect on stereoselectivity.

### Conversion rate to (1R,3R)-permethric acid and specificity for (1R,3R)-methyl permethemate

When the amount of product produced in the reaction catalyzed by the reference esterase from (1R,3R)-methyl permethrate to (1R,3R)-permethric acid was taken as 100%, the relative activity of the mutant enzymes was 159% for L151F/L242M/S329N and 157% for L151F/L242M/L200F.

The optical purity was 79% for the reference esterase, 88% for L151F/L242M/S329N, and 79% for L151F/L242M/L200F. Although all mutant enzymes showed higher conversion rates than the reference esterase, there was no effect on

### stereoselectivity.

### [Industrial Applicability]

By using the modified esterase of the present invention, (1R,3R)-CA, which is important as an intermediate for agricultural chemicals, can be efficiently produced. Therefore, the present invention is extremely useful, for example, in the field of agricultural chemical production.

This application is based on a patent application No. 2022-063122 filed in Japan (filing date: April 5, 2022), the contents of which are incorporated in full herein.

## Claims

1. A modified esterase of any of the following (i) to (iii):
(i) a modified esterase comprising an amino acid sequence in which at least one amino acid at a site selected from the group consisting of (1) to (24) below in the amino acid sequence of SEQ ID NO: 1 or 3 is substituted with another amino acid;
(1) V64
(2) S65
(3) A102
(4) A109
(5) L151
(6) I153
(7) Y173
(8) Y197
(9) L200
(10) 1211
(11) F217
(12) S220
(13) D222
(14) D227
(15) L229
(16) L242
(17) A245
(18) V257
(19) L298
(20) T301
(21) V326
(22) S329
(23) A350
(24) T371
(ii) a modified esterase in which one or more amino acids are further substituted (except for the amino acid site substituted in (i)), added, inserted, or deleted (except for the amino acid site substituted in (i)) in the modified esterase of (i), and the reactivity to (1R,3R)-methyl 3-(2-chloro-3,3,3-trifluoroprop-1-en-1-yl)-2,2-dimethylcyclopropane-1-carboxylate is improved compared to the reactivity of an esterase consisting of the amino acid sequence of SEQ ID NO: 1 or 3;
(iii) a modified esterase in which an amino acid(s) other than the amino acid(s) substituted in (i) is further substituted with another amino acid(s) in the modified esterase of (i), wherein the modified esterase has 70% or more identity with the modified esterase of (i) and has improved reactivity to (1R,3R)-methyl 3-(2-chloro-3,3,3-trifluoroprop-1-en-1-yl)-2,2-dimethylcyclopropane-1-carboxylate compared to the reactivity of the esterase consisting of the amino acid sequence of SEQ ID NO: 1 or 3.

2. The modified esterase according to claim 1, wherein in (i), the amino acid substitution is at least one selected from the group consisting of the following:
(1) V64M
(2) S65A
(3) A102S
(4) A109T
(5) L151F, L151M, or L151W
(6) I153V
(7) Y173F
(8) Y197F
(9) L200F
(10) I211V or I211M
(11) F217K
(12) S220W
(13) D222G
(14) D227C or D227E
(15) L229G
(16) L242C, L242I, L242M, or L242W
(17) A245T
(18) V257I
(19) L298H
(20) T301A
(21) V326I
(22) S329D, S329G, or S329N
(23) A350D
(24) T371A.

3. A nucleic acid encoding the modified esterase according to claim 1 or 2.

4. An expression cassette or recombinant vector comprising the nucleic acid according to claim 3.

5. A transformant transformed using the expression cassette or recombinant vector according to claim 4.

6. A method for producing a modified esterase, comprising the step of culturing the transformant according to claim 5.

7. An enzyme preparation comprising the modified esterase according to claim 1 or 2.

8. The enzyme preparation according to claim 7, which is used for producing (1R,3R)-3-[(1Z)-2-chloro-3,3,3-trifluoroprop-1-en-1-yl]-2,2-dimethylcyclopropane-1-carboxylic acid.

9. A method for producing (1R,3R)-3-[(1Z)-2-chloro-3,3,3-trifluoroprop-1-en-1-yl]-2,2-dimethylcyclopropane-1-carboxylic acid, comprising the step of acting the modified esterase according to claim 1 or 2 on (1R,3R)-methyl 3-(2-chloro-3,3,3-trifluoroprop-1-en-1-yl)-2,2-dimethylcyclopropane-1-carboxylate.
